# EUROPÄISCHE PATENTSCHRIFT

(11) **EP 1 447 053 B1**
(45) Veröffentlichungstag und Bekanntmachung des Hinweises auf die Patenterteilung: **11.07.2007**
(21) Anmeldenummer: 03405073.2
(22) Anmeldetag: 11.02.2003
(51) Int. Cl.: A61B 17/32

(54) **Instrument für Liposuction**
Instrument for liposuction
Dispositif de liposuction

(43) Veröffentlichungstag der Anmeldung: 18.08.2004
(73) Patentinhaber: Schaffer, Roland, 8008 Zürich (CH)
(72) Erfinder: Schaffer, Roland, 8008 Zürich (CH)
(74) Vertreter: Luchs, Willi

(56) Entgegenhaltungen:
- BE-A- 1 006 811
- US-A- 5 379 773
- US-A- 5 658 307
- US-A- 5 817 050
- US-A1- 2002 169 469

## Beschreibung

Die Erfindung betrifft ein Instrument für Liposuction, mit einem langgestreckten, stabförmigen Teil.

Liposuction bzw. Fettabsaugung ist heutzutage ein oft praktizierter medizinischer Eingriff. Eine Vorrichtung zum Durchführen eines solchen Verfahrens ist beispielsweise in der EP-B-0 458 989 offenbart. Diese Vorrichtung umfasst ein unter die Haut in die zu beseitigende Fettschicht cinführbares Absaugrohr. Das Hauptproblem bei der Fettabsaugung besteht darin, dass die Fettschicht oft ungleichmässig entfernt wird, wobei sich die Unregelmässigkeiten auch an der Hautoberfläche unästhetisch bemerkbar machen.

In der Druckschrift BE-A-1 006 811 ist ein Löffel mit einem stabförmigen Schaft dargestellt, welcher zum Abtrennen von Organen oder Gewebeteilen dient. Die Spitze dieses Löffels ist denn auch entsprechend als Messerstumpf ausgebildet. Auf der Unterseite des Löffels kann optionsweise eine abrasive Beschichtung vorgesehen sein, welche für das Trennen der Gewebeteile durch Abschleifen bestimmt ist. Die Form und die Spitze dieses Löffels sind dementsprechend messerartig ausgebildet. Es wäre somit gefährlich, einen solches Instrument mit einer frontseitigen Schneide für Liposuction einzusetzen.

Auch bei einem Verfahren zur Anwendung eines chirurgischen Trennungsinstrumente nach der Druckschrift US-A-5,658,307 ist ein stabförmiges Element mit einer gerundeten Spitze vorgesehen, welche mit abrasivem Material bestückt ist. Dieses abrasive Material dient wie eine mit Abrasivmaterial bestückte Trennschleifscheibe zur Trennung von Gewebe für chirurgische Zwecke. Dieses längliche Element wird hierzu für das Aufschneiden gedreht und quer zu seiner Längserstreckung bewegt.

In dem Dokument US-A-2002/0169469 ist eine Liposuction-Kanüle beschrieben, welche einen stabförmigen Teil und an seinem hinteren Ende einen Handgriff aufweist. Über einen Teil der Kanüle sind gleichmässig verteilte Vorsprünge am Umfang des rohrförmig ausgebildeten Stabes für ein Schaben des Gewebes vorgesehen, wobei diesen Vorsprüngen jeweils ein Öffnung für das Absaugen des abgeschabten Fettgewebes zugeordnet ist. Nachteilig bei dieser Kanüle ist die Tatsache, dass diese Kanüle steif ist und nicht den fettabzusaugenden Körperstellen angepasst werden kann, um Unregelmässigkeiten zu beheben. Zudem ist bei dieser Kanüle nicht eine gleichmässig aufgeraute Oberfläche vorgesehen.

Der vorliegenden Erfindung liegt die Aufgabe zugrunde, ein Instrument der eingangs genannten Art zu schaffen, welches einerseits ein Ausgleichen der beim Fettabsaugen entstehenden Unregelmässigkeiten ermöglicht und welches andererseits dem Problem überschüssiger Haut nach Liposuction entgegenwirkt.

Diese Aufgabe wird erfindungsgemäss durch die Merkmale des Anspruches 1 gelöst.

Weitere bevorzugte Ausgestaltungen des erfindungsgemässen Instrumentes bilden den Gegenstand der abhängigen Ansprüche.

Das erfindungsgemässe Instrument kann während der Liposuction abwechslungsweise mit einem Absaugeinstrument eingesetzt werden, wobei sein langgestreckter, stabförmiger Teil teilweise unter die Haut zu der bereits mit dem Absaugeinstrument behandelten Stelle eingeführt und dort - durch eine Hin-und Herbewegung in seiner Längsrichtung - mit seiner aufgerauhten Oberfläche die verbliebenen Fettschicht-Reste gleichmässig verteilen oder vom übrigen Gewebe lösen kann, die dann anschliessend wieder mit dem Absaugeinstrument entfernt werden können. Zudem wird dem Problem überschüssiger Haut nach Liposuction entgegenwirkt, indem die Haut durch mechanische Induktion einer feinen kontrahierenden Bindegewebeschicht gestrafft wird.

In einer besonders bevorzugten Ausführung besteht der stabförmige Teil aus rostfreiem, elastisch biegsamen Stahl, der eine Anpassung der Stabform an die zu behandelnden Körperteile erlaubt und eine besonders vorteilhafte, einfache Handhabung ermöglicht.

Die Erfindung wird nachfolgend anhand der Zeichnung näher erläutert. Es zeigen:
- Fig. 1: ein Ausführungsbeispiel eines erfindungsgemässen Instrumentes in Seitenansicht; und
- Fig. 2: das Instrument nach Fig. 1 in Draufsicht.

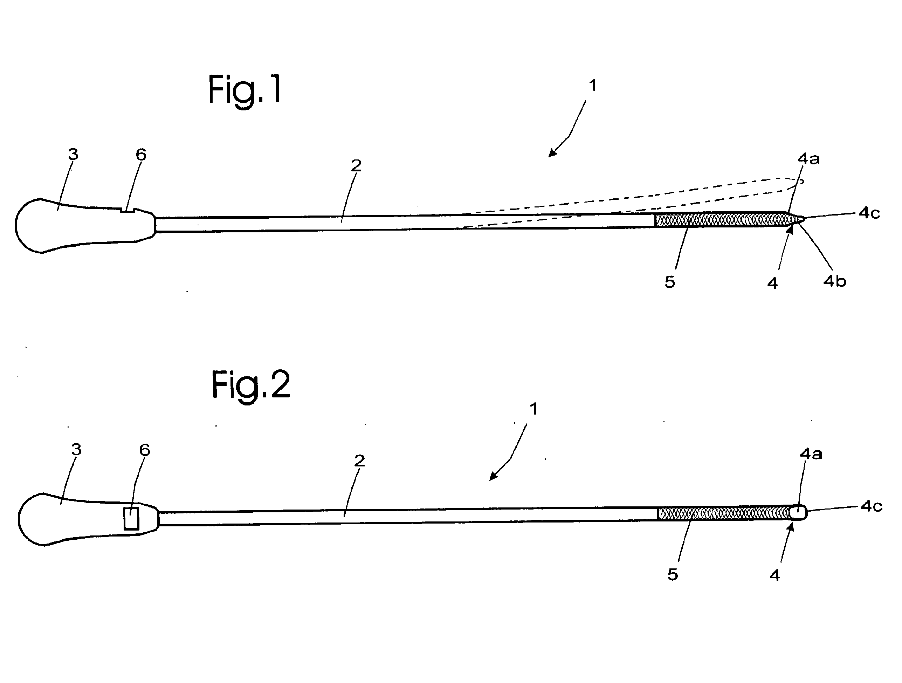

In Fig.1 und 2 ist ein bei Liposuction verwendbares Instrument 1 dargestellt, das einen langgestreckten, stabförmigen Teil 2 aufweist, der an seinem hinteren Ende mit einem Handgriff 3 versehen ist. Der stabförmige Teil 2 ist im Querschnitt vorzugsweise rund, könnte aber auch beispielsweise eine ovale oder gerundete Kanten aufweisende Querschnittform besitzen. An seinem vorderen Ende ist der stabförmige Teil 2 mit einem Endstück 4 ausgestattet, das eine obere Fläche 4a sowie eine untere Fläche 4b aufweist, die zu einer vorderen Kante 4c zusammenlaufen. Die vordere Kante 4c ist sowohl in ihrem Querschnitt (vgl. Fig. 1), als auch in ihrem Verlauf (vgl. Fig. 2) gerundet.

Während der Handgriff 3 vorzugsweise aus Kunststoff hergestellt ist, besteht der stabförmige Teil 2 mit Vorteil aus rostfreiem, elastisch biegsamen Stahl. Dank der elastischen Biegsamkeit kann die Form des Instruments 1 bzw. des Teiles 2 verändert werden (von Hand), wie in Fig. 1 gestrichelt angedeutet. In einem an das vordere Endstück 4 anschliessenden vorderen Bereich weist der stabförmige Teil 2 eine aufgerauhte Oberfläche 5 auf, die beispielsweise durch Kordeln oder Rändeln angefertigt wird (die aufgerauhte Oberfläche könnte sich auch weiter nach hinten erstrecken oder es könnten mehrere aufgerauhten Flächen hintereinander angeordnet sein). Die Endstück-Flächen 4a, 4b sind jedoch nicht aufgerauht (und auch die Kante 4c nicht).

Der Handgriff 3 ist mit einer Referenzfläche 6 versehen, die parallel zu der vorderen Kante 4c des Endstückes 4 verläuft.

Das erfindungsgemässe Instrument 1 kann mit dem Endstück 4 und teilweise mit dem stabförmigen Teil 2 annähernd parallel zur Hautoberfläche unter die Haut zu der zu behandelnden Stelle eingeführt und dort in seiner Längsrichtung hin und her bewegt werden, um mit der aufgerauhten Oberfläche 5 auf die Fettschicht einzuwirken und sie vom übrigen Gewebe zu lösen. Das Instrument 1 wird dabei so gehalten, dass die vordere Kante 4c (und beim ungebogenen Teil 2 auch die Referenzfläche 6) annähernd parallel zur Hautoberfläche verläuft.

Bei der Liposuction wird das Instrument 1 als Hilfsinstrument abwechslungsweise mit einem an sich bekannten Absaugeinstrument eingesetzt, wobei in der Regel zuerst das Absaugen der Fettschicht erfolgt, wonach mittels des erfindungsgemässen Instrumentes 1 die verbliebenen Fettschicht-Reste vom übrigen Gewebe gelöst und die Unregelmässigkeiten sozusagen abgerieben werden, und anschliessend das Fett wieder mit dem Absaugeinstrument entfernt wird. Durch die Formgestaltung des vorderen Endstücks 4 mit gerundeter Kante 4c, ohne Kanten und Ecken, werden Haut- und Gewebeverletzungen vermieden.

Ein aus elastisch biegsamem Material bestehender stabförmiger Teil 2 erlaubt eine Anpassung der Stabform an die zu behandelnden Körperteile, wodurch eine besonders vorteilhafte, einfache Handhabung ermöglicht wird.

Es wäre theoretisch möglich, die aufgerauhte Oberfläche zur Beseitigung der beim Fettabsaugen entstehenden Unregelmässigkeiten direkt auf einem hohlstabförmig ausgebildeten, unter die Haut einführbaren, langgestreckten Absaugeinstrument anzuordnen.

## Patentansprüche

1. Instrument für Liposuction, mit einem langgestreckten, stabförmigen Teil (2), welcher an seinem hinteren Ende mit einem Handgriff (3) versehen ist und ein vorderes Endstück (4) zum teilweisen Einführen des stabförmigen Teiles (2) unter die Haut zu der zu behandelnden Stelle aufweist, wobei der stabförmige Teil (2) zumindest über einen Teil seiner Länge eine aufgeraute Oberfläche (5) aufweist, wobei der stabförmige Teil (2) von Hand elastisch biegsam ausgebildet ist und vorzugsweise aus rostfreiem Stahl besteht, wobei das vordere Endstück (4) eine obere sowie eine untere Fläche (4a, 4b) aufweist, die zu einer vorderen Kante (4c), welche sowohl in ihrem Querschnitt als auch in ihrem Verlauf gerundet ist, zusammenlaufen und diese obere sowie die untere Fläche (4a, 4b) symmetrisch zur Längsachse des stabförmigen Teils (2) angeordnet sind, wobei das Instrument (1) mit dem Endstück (4) und teilweise mit dem stabförmigen Teil (2) annähernd parallel zur Hautoberfläche unter die Haut zu der zu behandelnden Stelle einführbar und dort in seiner Längsrichtung hin und her bewegbar ist, um mit der aufgerauten Oberfläche (5) auf die Fettschicht einzuwirken und sie vom übrigen Gewebe zu lösen.

2. Instrument nach Anspruch 1, **dadurch gekennzeichnet, dass** der stabförmige Teil (2) aus rostfreiem Stahl besteht.

3. Instrument nach Anspruch 1 oder 2, **dadurch gekennzeichnet, dass** der stabförmige Teil (2) in einem an das vordere Endstück (4) anschliessenden vorderen Bereich mit der aufgerauhten Oberfläche (5) versehen ist.

4. Instrument nach Anspruch 1, **dadurch gekennzeichnet, dass** der Handgriff (3) mit einer Referenzfläche (6) versehen ist, die parallel zu der vorderen Kante (4c) verläuft.

5. Instrument nach einem der Ansprüche 1 bis 4, **dadurch gekennzeichnet, dass** der stabförmige Teil (2) eine runde, ovale oder gerundete Kanten aufweisende Querschnittform aufweist.

6. Instrument nach einem der Ansprüche 2 bis 5, **dadurch gekennzeichnet, dass** die aufgeraute Oberfläche (5) durch Kordeln oder Rändeln hergestellt ist.

## Claims

1. Instrument for liposuction with, an elongated, rod-shaped part (2), which is equipped with a handle (3) at its rear end and incorporates a front end piece (4) for partial insertion of the rod-shaped part (2) under the skin of the location that is to be treated, whereby the rod-shaped part (2) incorporates a roughened surface (5) at least along parts of its length, wherein the rod-shaped part (2) is especial of stainless steel and produced to be elastically bent by hand, whereas the front end piece (4) incorporates an upper as well as lower surface (4a, 4b) which meet to form a front edge (4c), whereby the cross-section as well as the expansion of the edge (4c) is rounded, and this upper as well as lower surface (4a, 4b) is arranged symmetrically to the longitudinal axis of the rod-shaped part (2), whereby the instrument (1) with the end piece (4) and a part of the rod-shaped part (2) can be inserted under the skin at the location that is to be treated almost parallel with the skin surface and moved backwards and forwards there in a longitudinal direction in order to treat the fat layer with the roughened surface (5) and to separate the same from the underlying tissue.

2. Instrument according to claim 1, **characterised in that** the rod-shaped part (2) consists of stainless steel.

3. Instrument according to Claim 1 or 2, **characterised in that** the rod-shaped part (2) is equipped with a front area incorporating the roughened surface (5) adjoining the front end piece (4).

4. Instrument according to Claim 1, **characterised in that** the handle (3) is equipped with a reference surface (6), which extends parallel with the front edge (4c).

5. Instrument according to one of the claims 1 to 4, **characterised in that** the rod-shaped part (2) incorporates a round, oval or rounded edge cross-section.

6. Instrument according to one of the claims 2 to 5, **characterised in that** the roughened surface (5) is manufactured by cutting or milling.

## Revendications

1. Instrument de liposuccion comprenant une partie (2) en forme de barre qui s'étend en longueur et qui est munie d'une poignée (3) à son extrémité arrière et qui a une pièce (4) d'extrémité avant pour introduire partiellement la partie (2) en forme de barre sous la peau, à l'emplacement à traiter, la partie (2) en forme de barre ayant une surface (5) rugueuse au moins sur une partie de sa longueur, la partie (2) en forme de barre étant constituée de façon à pouvoir être courbée élastiquement à la main, et de préférence, en acier inoxydable, la pièce (4) d'extrémité avant ayant une surface (4a) supérieure ainsi qu'une surface (4b) inférieure qui se rejoignent en une arête (4c) avant arrondie tant dans sa section transversale que dans son tracé et ces surfaces (4a, 4b) supérieure et inférieure sont disposées de façon symétrique par rapport à l'axe longitudinal de la partie (2) en forme de barre, l'instrument (1) pouvant être introduit sous la peau à l'emplacement à traiter à peu près parallèlement à la surface principale par la pièce (4) d'extrémité et en partie par la partie (2) en forme de barre et pouvant y être déplacé suivant un mouvement de va-et-vient dans sa direction longitudinale pour agir par la surface (5) rugueuse sur la couche de graisse et la détacher du tissu restant.

2. Instrument suivant la revendication 1, **caractérisé en ce que** la partie (2) en forme de barre est en acier inoxydable.

3. Instrument suivant la revendication 1 ou 2, **caractérisé en ce que** la partie (2) en forme de barre est munie de la surface (5) rugueuse dans une zone avant se raccordant à la pièce (4) d'extrémité avant.

4. Instrument suivant la revendication 1, **caractérisé en ce que** la poignée (3) est munie d'une surface (6) de référence qui s'étend parallèlement à l'arête (4c) avant.

5. Instrument suivant l'une des revendications 1 à 4, **caractérisé en ce que** la partie (2) en forme de barre a une forme de section transversale circulaire ou ayant des arêtes ovales ou arrondies.

6. Instrument suivant l'une des revendications 2 à 5, **caractérisé en ce que** la surface (5) rugueuse est produite par moletage ou cordonnage.
